(19) **Europäisches Patentamt / European Patent Office / Office européen des brevets**

(11) **EP 3 618 702 B1**

(12) **EUROPEAN PATENT SPECIFICATION**

(45) Date of publication and mention of the grant of the patent:
**18.09.2024 Bulletin 2024/38**

(21) Application number: **18721335.0**

(22) Date of filing: **26.04.2018**

(51) International Patent Classification (IPC):
**A61B 5/024** $^{(2006.01)}$  **A61B 5/00** $^{(2006.01)}$
**G06T 7/00** $^{(2017.01)}$  **A61B 5/1455** $^{(2006.01)}$
**A61B 5/1171** $^{(2016.01)}$  **A61B 5/08** $^{(2006.01)}$

(52) Cooperative Patent Classification (CPC):
**A61B 5/02416; A61B 5/0077; A61B 5/7207; A61B 5/7257; G06V 40/171;** A61B 5/0816; A61B 5/1176; A61B 5/1455; A61B 5/7246; A61B 5/725; A61B 2576/00; G06F 2218/16; G06V 40/15; G16H 30/40

(86) International application number:
**PCT/EP2018/060674**

(87) International publication number:
**WO 2018/202521 (08.11.2018 Gazette 2018/45)**

(54) **A SYSTEM AND METHOD FOR EXTRACTING A PHYSIOLOGICAL INFORMATION FROM VIDEO SEQUENCES**

SYSTEM UND VERFAHREN ZUM EXTRAHIEREN VON PHYSIOLOGISCHEN INFORMATIONEN AUS VIDEOSEQUENZEN

SYSTÈME ET PROCÉDÉ PERMETTANT D'EXTRAIRE DES INFORMATIONS PHYSIOLOGIQUES À PARTIR DE SÉQUENCES VIDÉO

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.05.2017 EP 17169542**

(43) Date of publication of application:
**11.03.2020 Bulletin 2020/11**

(73) Proprietor: **Koninklijke Philips N.V.**
**5656 AG Eindhoven (NL)**

(72) Inventors:
• **DEN BRINKER, Albertus, Cornelis**
  **5656 AE Eindhoven (NL)**
• **BULUT, Murtaza**
  **5656 AE Eindhoven (NL)**
• **JEANNE, Vincent**
  **5656 AE Eindhoven (NL)**
• **ASSELMAN, Michel, Jozef, Agnes**
  **5656 AE Eindhoven (NL)**
• **KERSTEN, Gerrit, Maria**
  **5656 AE Eindhoven (NL)**

(74) Representative: **Philips Intellectual Property & Standards**
  **High Tech Campus 52**
  **5656 AG Eindhoven (NL)**

(56) References cited:
**WO-A1-2016/193735  WO-A2-2006/091636**
**US-A1- 2006 293 575**

• **PRATHOSH A P ET AL: "Estimation of Respiratory Pattern From Video Using Selective Ensemble Aggregation", IEEE TRANSACTIONS ON SIGNAL PROCESSING, IEEE SERVICE CENTER, NEW YORK, NY, US, vol. 65, no. 11, 2 February 2017 (2017-02-02), pages 2902 - 2916, XP011644307, ISSN: 1053-587X, [retrieved on 20170328], DOI: 10.1109/TSP.2017.2664048**

**Description**

FIELD OF THE INVENTION

The present invention relates to the extraction of information from video sequences and, in particular to the extraction of physiologically-related information (often referred to as vital signs).

BACKGROUND

It is possible to analyze video sequences of a living subject and detect small changes in the images which are the result of physiological processes of that subject. Amongst these physiological process are such things as blood flow, breathing and sweating. An example for this method can be found in PRATHOSH A P ET AL: "Estimation of Respiratory Pattern From Video Using Selective Ensemble Aggregation", IEEE TRANSACTIONS ON SIGNAL PROCESSING, IEEE SERVICE CENTER, NEW YORK, NY, US, vol. 65, no. 11, 2 February 2017 (2017-02-02), pages 2902-2916.

Certain physiological processes can be observed via skin reflectance variations. The human skin can be modelled as an object with at least two layers, one of those being the epidermis (a thin surface layer) and the other the dermis (a thicker layer underneath the epidermis). A certain percentage 5 % of an incoming ray of light is reflected at the skin surface. The remaining light is scattered and absorbed within the two skin layers in a phenomenon known as body reflectance (described in the Dichromatic Reflection Model). The melanin, typically present at the boundary of epidermis and dermis, behaves like an optical filter, mainly absorbing light. In the dermis, light is both scattered and absorbed. The absorption is dependent on the blood composition, so that the absorption is sensitive to blood flow variations. The dermis contains a dense network of blood vessels, about 10 % of an adult's total vessel network. These vessels contract and expand according of the blood flow in the body. They consequently change the structures of the dermis, which influences the reflectance of the skin layers.

Other physiological processes such as breathing cause movement in the surface of patient.

Other physiological processes such as variations in blood oxygenation level can manifest themselves as small colour changes.

It is possible to detect and extract signals which have some periodic content in these changes and from that obtain a result such as a frequency in the case of periodic processes. For example, a subject may be illuminated with ambient light and filmed using a video camera. By analyzing changes in the values of corresponding pixels between frames of the sequence of images, a time-variant signal can be extracted. This signal may be transformed into frequency-like domain using something like a Fast Fourier Transform and from the frequency-domain spectra, a value for the subject's heart-rate may be arrived at as a physiological measurement. These physiological measurements are often called vital signs.

The changes in the pixel values are often small and often more pronounced in 1 colour channel than the others. Thus the signal that is being looked for is correspondingly small.

There may be other changes in the pixel values such as those due to changes in the general image and these can be large in comparison to the signal. There are also sources of random change in the pixel values such as movement of the subject, changes in the illumination (such as flicker) and noise in the image sensor and variations in the illumination. All of these are, to all intents and purposes, uncorrelated with the signal being sought. Therefore the signal to noise ratio is small and it may be difficult to obtain a meaningful physiological measurement.

SUMMARY OF THE INVENTION

There is provided a method of extracting a physiological information representing a vital sign of a subject, the method comprising, for a defined time-period, receiving a sequence of video images of the subject, extracting a plurality of signal segments in a time-domain form from a plurality of respective patches in images in the sequence of video images, selecting a subset of the plurality of signal segments wherein selecting comprises rejecting segments having a high maximum signal level, converting at least said subset to produce a plurality of transformed signal segments, and combining the at least said subset of the plurality of transformed signal segments to produce a combined signal segment and finding a dominant frequency or periodic component in the combined signal segment, the dominant frequency or periodic component being representative of the physiological information.

This has the advantage of reducing the influence of motion and or illumination-induced changes on the final result by removing parts of the raw signal segments which are worst affected by these.

According to an embodiment, the method comprises rejecting segments having a maximum signal level greater than a threshold. This exploits the fact that the signal of interest which contains the physiological information is small and so large signals can be considered as being due to motion or illumination changes.

According to an embodiment, the selecting comprises weighting segments with weights having an inverse relationship to their maximum signal level. This has the advantage of not requiring a hard threshold which may be difficult to set satisfactorily.

According to an embodiment, the inverse relationship is of the form of a power law or an inverse exponential which has the advantage of providing a strong selectivity for small signal levels as compared to large signal levels.

According to an embodiment, the conversion is performed using one of a power spectral density function, an auto-correlation function or a Fourier transform which provide the signal in a frequency-like domain from which the physiological information, which is in the form of a frequency, may be extracted.

According to an embodiment, the combining of the signal segments comprises averaging the signal segments which affords a signal which had further reduced levels of unwanted components.

According to an embodiment, method further comprisises epeating at least once the method described above, storing the combined signal segment at each repetition to produce a plurality of combined signal segments, and averaging the plurality of combined signal segments to produce a final combined signal. This affords further reduction of unwanted components

According to an embodiment, the settings for the averaging of the plurality of combined signal segments are derived from a previously stored combined signal segment. This allows for adaptive filtering or averaging which helps reduce unwanted components.

According to an embodiment, the method further comprises performing an interpolation of the combined signal which provides further smoothing an unwanted component reduction.

According to an embodiment, the method further comprises extracting a value representative of the physiological information which may then be provided as an output.\

According to an embodiment, the method further comprises acquiring a sequence of video frames and the extracting of the plurality of signal segments is performed using remote photoplethysmography. This has the advantage of affording a non-contact (or non-invasive) method of acquiring the signals for analysis and may be performed using a video-camera.

There is provided a system for extracting a physiological information representative of a vital sign comprising an input configured to receive a sequence of video images of the subject, a signal extraction unit configured to, for a defined time-period, extract a plurality of signal segments in a time-domain form from a plurality of respective patches in images in the sequence of video images, a selection unit configured to select a subset of the plurality of signal segments wherein the selection comprises rejecting segments having a high maximum signal level, a convertion unit configured to convert at least said subset to produce a plurality of transformed signal segments, and a combining unit configured to combine the at least said subset of the plurality of transformed signal segments to produce a combined signal segment and a physiological measurement extractor (28) configured to find a dominant frequency or periodic component in the combined signal segment, the dominat frequency or periodic component being representative of the physiological information.

According to an embodiment, the combining unit of the system further comprises a time-averaging unit configured to store the combined signal segments, to store interpolation settings, to apply those stored settings to interpolate subsequent combined signal segments and to combine the stored combined signal segments. This allows reduction of unwanted components by averaging over a longer period of time.

There is provided a computer program software product, stored on a computer-readable medium, configured, when executed on a processor, to execute method described herein. This allows a general purpose computer, when linked to a video camera to operate the method described herein.

There is provided a physiological measurement equipment comprising a system as described herein.

BRIEF DESCRIPTION OF THE DRAWINGS

The above, as well as additional objects, features and advantages of the disclosed systems and methods, will be better understood through the following illustrative and non-limiting detailed description of embodiments of devices and methods, with reference to the appended drawings, in which:

Fig. 1 represents a setup according to an embodiment for measuring a physiological information of a subject.

Fig.2 represents a video sequence processing chain according to an embodiment.

Fig. 3 represents a case of performing the measurement according to an embodiment.

Fig. 4 represents a process according to an embodiment of extracting a physiological information.

Fig 5 represents a curve of a weight according to an embodiment for application to the amplitudes of a signal segment.

Fig. 6 represents a flow of a method according to an embodiment.

DETAILED DESCRIPTION OF THE EMBODIMENTS

In the following description, same references designate like elements.

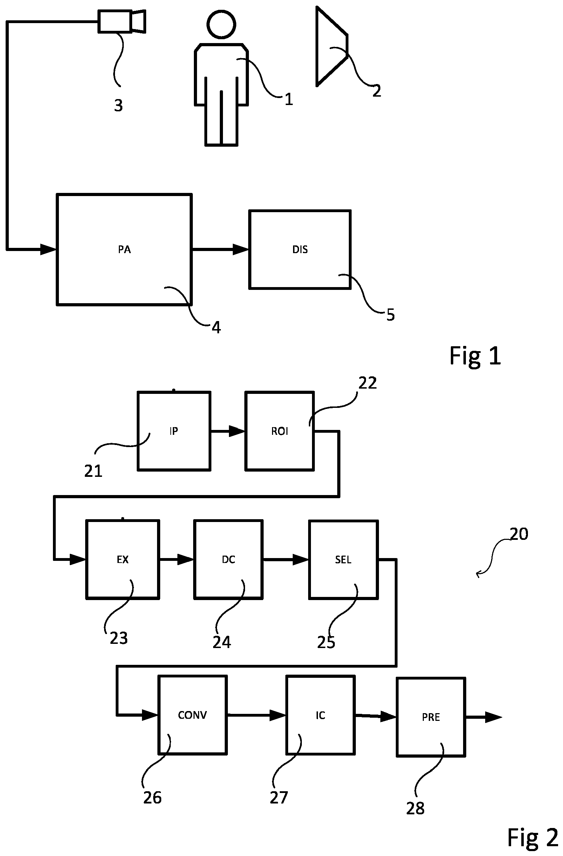

Fig. 1 represents a setup for measuring a physiological process of a subject 1 and extracting a physiological information or vital sign. A light source 2 (which may be artificial or natural) illuminates the subject 1. A video camera 3 records a sequence of video frames and feeds them to a processing unit (PA) 4 which, extracts the vital sign and in turn provides an output to a display 5. The display 5 may display just the physiological information from the signal analysis unit 4 either alone or in combination with the video sequence.

Fig. 2 represents a processing chain 20 according to an embodiment and configured to extract the physiological information. The processing chain 40 may be conveniently implemented as part of the processing device 4. An input (IP) 21 receives the video sequence and passes the frames of the video sequence to a patch selecting unit 22 (ROI) which selects the patches or ROIs in the images of the video sequence that are to be tracked. There may be one or more patches which are selected for subsequent processing. The patch selecting unit 22 feeds a series patches to a signal extractor 23 (EX). The signal extractor 23 performs operations on the signal in order to arrive at the time-varying signal of interest. These operations may include the combining of the colour channels and/or the normalizing of the signals. It may be advantageous to perform motion compensation and so it may be that the sequence of patches has been broken up into shorter sequences in order to make the task of motion compensation easier. The extracted time-varying signals are then fed to a decomposition unit (DC) 24 which performs a decomposition of the signals so as to be able to remove the DC component and perform some cleaning. The cleaning may involve removal of high-frequent noise (by low-pass filtering), removal of disturbing components (e.g. spikes by median filtering), or rejection of signals with large signal discontinuities.Then the cleaned signals are fed to a signal selection unit (SEL) 25 which selects amongst the signals those which will be used for further processing. A conversion unit (CONV) 26 performs a transformation of the time-varying signals so as to allow extraction of a periodic property of the signal, the periodic property being representative of the physiological information or vital sign. The transformed signals are then fed to a combining unit (IC) 27 which combines the transformed signals into a single signal and feeds this single signal to a physiological measurement extractor (PRE) 28 which extracts the physiological measurement from this single signal.

Thus there is method of extracting a physiological information over a time-period which comprises extracting a plurality of signal segments in a time-domain form, selecting a subset of the plurality of signal segments according their maximum signal level and then converting at least said subset to produce a plurality of transformed signal segments, and then combining the at least said subset of the plurality of transformed signal segments to produce a combined signal segment.

The processing chain 20 may be implemented in one or more general purpose processors running appropriate software. This has the advantage of being possible with pre-existing hardware and allows for subsequent modification and tuning. However it can result in a solution which is slower and/or more expensive than a mode dedicated solution. Alternatively some or all of the individual components may be implemented in microcontrollers running firmware designed to implement the relevant functions. This solution may be less expensive when production volumes are sufficiently high enough. Yet another possibility is to implement the functions in dedicated hardware. In high volumes, this is often cheaper and gives higher processing speed per unit cost.

The patches are selected using one or more of a number of methods. A process which is sometimes called 'segmentation' is performed. It is convenient to start by selecting the general area of interest. The face is suitable whenever blood flow is the physiological process of interest so a face-identification algorithm may be used. A suitable algorithm for implementing face detection is described in Viola, P. and Jones, M.J., "Robust real-time object detection", Proc. of IEEE workshop on statistical and computational theories of vision, 13 July 2001. Alternative algorithms for recognizing shape and colour patterns also exist and these may be used for detecting the facial area. For other processes like breathing, other methods for identifying the thorax may be used.

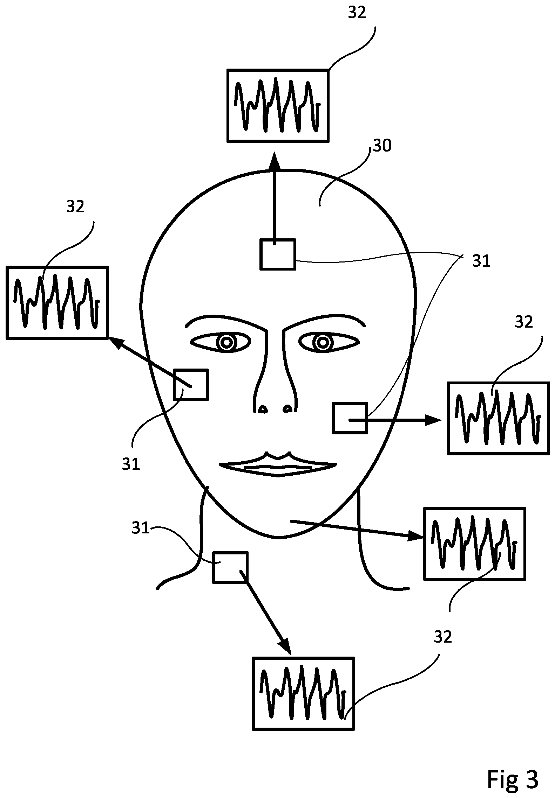

Fig. 3 represents an exemplary situation where a selected region 30 (in this case a face of a subject 1) is being used. A plurality of patches 31 has been selected from which time-varying signals 32 have been extracted over a period of time. Whilst the time-varying signals 32 have been extracted at nominally the same time, they are not perfectly synchronized. Therefore, whilst these time varying signals 32 contain the same physiological information, it is not effective to combine them in the time-domain. Also, as mentioned previously, for the purposes of motion compensation, the time-varying signal 32 from each patch 31 may be collected as a series of signal segments over a longer period of time, with the objective of combining these signal segments into a single signal.

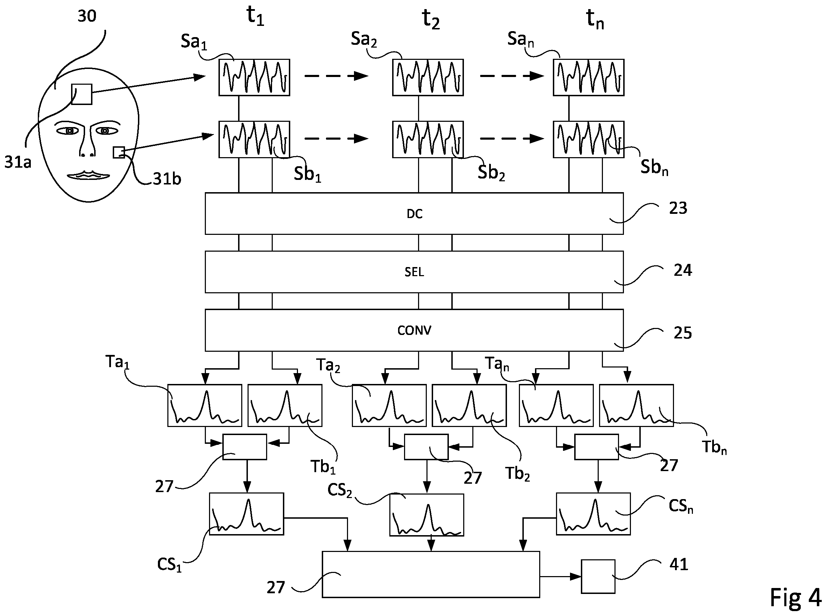

Fig. 4 represents a process or method according to an embodiment, as described above but in more detail. From a plurality of patches 31a, 31b on the selection region 30, over a series of time slots $t_1$, $t_2$, $t_3$, a plurality of series of time-varying signals segments $Sa_{1-n}$, $Sb_{1-n}$ are extracted and collected. Each of the time-varying signals $Sa1-n$, $Sb1-n$ is individually processed by a decomposition unit 24 which performs DC removal and cleaning. From the individual time-varying signals segments $Sa_{1-n}$, $Sb_{1-n}$, the selecting unit 25 selects the preferred signal segments that will ultimately combined for extraction of the physiological information.

The selection is performed so as to remove those signal segments which would degrade the overall signal-noise-ratio. The inventors have found that a significant source of problems are those signal segments that come from patches where motion of the subject 1 or variations in the illumination are observable and that these patches can be identified by the amplitude of the time-varying signal extracted therefrom. The inventors have made the surprising observation that amplitudes which are too great are indicative of the presence of problems, particularly motion or illumination-variation artifacts. This is somewhat counter intuitive in that normally in a search for greater signal-to-noise ratios, the skilled person would prefer greater signal amplitudes and reject those with smaller amplitudes. There are various embodiments for the selection process. Firstly, the patches may be assessed in a number of ways. The standard deviation of the pixel values in the patch over the time period concerned may be used because this is related to the amplitude of the time-varying signal extracted therefrom. Another possibility is to assess the total power or energy of the signal which is given by the variance of the pixel values of the patch over time. These have the advantage of being easy to integrate into the signal processing chain since such functions are also used for the motion tracking. A further possibility is to assess the maximum signal amplitude in the actual time-varying signal segments, which is more direct and may be easier to tune but requires extra functions and processing. In the case where the selection is performed by examining the pixel values of the patch, the selection could be made before signal extraction which could save some processing power.

Next the results of the assessment are used to make a selection. This selection can be considered as rejecting signal segments having higher maximum signal levels in favour of those having lower maximum signal levels. This can be selecting a maximum threshold value and rejecting all cases exceeding this. An alternative is to apply a weighting to the individual time-varying signal segments where the weightings have an inverse relationship to the signal amplitude (measured or inferred). It is also possible to use a relationship which is not linear but has a power law form like

$$f(x) = ax^{-k} \qquad [1]$$

or an inverse exponential form like

$$f(x) = ae^{-bx} \qquad [2]$$

The linear scaling factor a, the power law exponent and the exponential scaling factor may be adjusted to give the desired results.

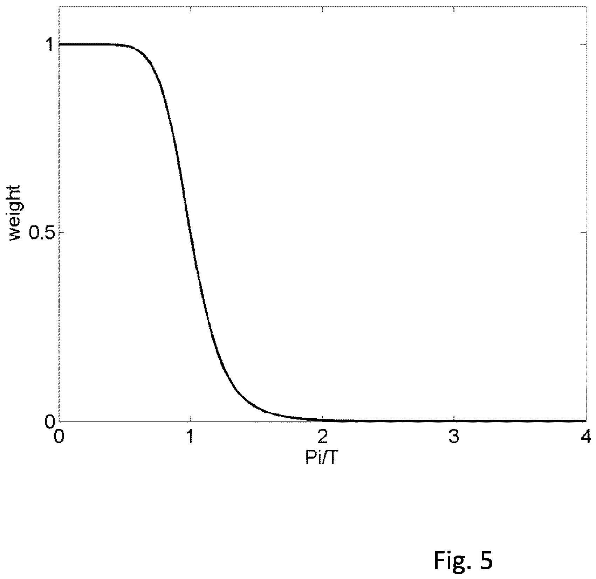

Fig. 5 represents a curve showing the result of two convenient ways of applying weightings. It shows the value of the weight versus the ratio of a threshold over an amplitude. These ways have the following relationships:

$$w(T:P_i) = \frac{T^2}{(T^2 + P_i^2)} \qquad [3]$$

and

$$w(T:P_i) = \frac{T^4}{(T^4 + P_i^4)} \qquad [4]$$

where T is the threshold and $P_i$ is a feature of the signal segment reflecting the amplitude of the i-th signal, from a group $S_i$ of 1 to n signal segments. For amplitudes $P_i$ below the threshold T, the weight is substantially equal to 1. For amplitudes above T, the weight shows a strong decrease as a function of the amplitude. The feature $P_i$ may be statistical characteristic of the pixel values over time, such as the standard deviation or the total power of the signal as given by the variance of the pixel values over time.

The threshold may be set by a calibration routine run at manufacturing time. This has the advantage of being simple to implement and lower cost. Another method is to arrange for the system to run a training routine or sequence where the threshold is set by checking the actual vital signs result and adjusting the threshold accordingly. This adjustment could be under the control of a technician or indeed under automatic control of the system itself. Where automatic control is used, the adjustments routine could be arranged to run over a long period of use or until the system finds that the threshold value obtained is no longer varying.

Using this weighting technique, particularly with a non-linear function has the advantage that it allows the rejection of the outlying i.e. very large values whilst reducing the difficulty of accurately determining the best parameters (which might vary between situations or equipment characteristics) in that they operate more 'softly'. Nevertheless, it is also possible to combine selection methods such as using a threshold and then a linear and/or non-linear weighting methods. And advantage of this could be computation speed in that very extreme results could be excluded without the need to calculate the weightings, thereby saving time and computational effort.

The selected time-varying signal segments $Sa_{1-n}$, $Sb_{1-n}$ are then transformed by the conversion unit 25 into transformed signal segments $Ta_{1-n}$, $Tb_{1-n}$, of a form from which a dominant frequency or periodicity can be extracted. Such operations could be transforming into a spectrum, i.e. frequency domain, by using something like a Discrete or Fast Fourier Transform (DFT or FFT). From the spectrum the DC component and other components considered out-of-band may be discarded and a peak corresponding to the fundamental frequency of the pertinent physiological process.

A DFT may be expressed as, for a sequence of N complex numbers $x_n$

$$X_k \stackrel{\text{def}}{=} \sum_{n=0}^{N-1} x_n \cdot e^{-j2\pi kn/N} \qquad [5]$$

Another method could be to use an autocorrelation or ACF (also sometimes known as a cross-autocorrelation or serial correlation) function to arrive at result indicative of a quasi-periodic signal. By way of illustration only, a common formation of an estimation for autocorrelation function for a signal for which n observations have been made and for which there are mean $\mu$ and variance $\sigma^2$ :

$$\hat{R}(k) = \frac{1}{(n-k)\sigma^2} \sum_{t=1}^{n-k} (X_t - \mu)(X_{t+k} - \mu) \qquad [6]$$

where k is an integer less than n.

From the inverse of the time lag between the peaks of the autocorrelation, a frequency of the periodic signal can be derived.

A third method could be to use a power spectral density function (PSDF). This function represents the frequency distribution of the power of a signal. It is sometimes defined or expressed as, for a finite time series $x_n$ of samples of a signal, the samples being at discrete times $x_n = x(n\Delta t)$ for a total time period T= N$\Delta t$:

$$S_{xx}(\omega) = \frac{(\Delta t)^2}{T} \left| \sum_{n=1}^{N} x_n \, e^{-i\omega n} \right|^2 \qquad [7]$$

where n is between 1 and N

It may be useful to vary the above expressions or choose different formulations, possibly with other terms, when implementing them in a system for extracting physiological information.

Another possibility is a Laplace transform which can also be used to obtain a frequency-domain representation of a signal from its time-domain form.

Other possibilities exist such as the multiple signal classification (MUSIC) algorithm, the pitch detection algorithm (PDA), the average magnitude different function (ADMF), the average squared mean difference function (ASDMF). There are also algorithms known as the YIN algorithm and the MPM algorithms respectively.

It may also be possible to use multiple transform methods. This would allow comparison of the results for the purposes of confirmation and judging the quality of the result. In a situation where the quality was too low, a reselection with tighter criteria (for example, a lower threshold, coefficients adjusted to give higher non-linearity) could be performed.

It should be noted that the step of selecting signal segments according to their maximum amplitude could also be performed in the frequency domain i.e. after transformation. This would have the advantage of simplifying the selection a little at the expense of extra computational effort transforming segments that would otherwise have been rejected.

Next the transformed signal segments $Ta_{1-n}$, $Tb_{1-n}$ are combined by the combining unit 27 into combined signal segments $CS_{1-n}$. At this point, the combined signal segments $CS_{1-n}$ still correspond to the time periods $t_{1-n}$ from which they came, within the limits that some elimination may have occurred during the selection procedure described previously. It should be understood that the combination, which can be averaging the respective signal segments together, is now possible because the transformation has removed the relative phase information that prevented combination of the time-domain representations. This has the advantage of reducing still further unwanted components such as noise, distortions or artefacts introduced by the conversion process.

The combined signal segments $CS_{1-n}$, which have been collected over time, are further combined into a final combined signal 41 from which the physiological information can be derived by finding a dominant frequency or periodic component. In this combining step, noise may be reduced by (weighted) averaging over combined signal segments CSn, collected over a period of time. This can be considered as executing an identical temporal filter per element of signal segments. This filter may be implemented as an FIR or IIR filter.FIR and IIR filters will give different results and the choice between them is made according to different constraints - for example a requirement on linear phase could lead more toward an FIR. Such a choice is within the reach of the skilled person. It is advantageous to perform interpolation to increase the resolution. Examples of suitable methods of interpolation are linear, polynomial or spline. Both steps can be combined and thus the combination unit acts as a time-averaging unit which is configured to store the combined signal segments, to store interpolation values, to store the filter states, and to combine the combined signal elements. The filter settings may be adaptively controlled. If filters with a short time constant already provide a clear indication of the physiological feature of interest (e.g., the frequency or repetition rate of the blood volume pulse) one may select and use this setup. However, if the feature of interest does not stand out sufficiently in the output signal, a larger time constant (i.e. other filter coefficients) can be chosen. The decision method may be as simple as a comparing values obtained for the feature to a threshold. For example, in case where the feature is a frequency associated with the highest peak in the Fourier transform, one may determine the peak value relative to the background level (environment) and let that serve as a control variable. Thus the filter settings are controlled by a local contrast measure within the results output by the function being used. A similar mechanism can be operated when searching for a delay in an ACF. Using a smaller time constant gives the advantage of obtaining a result more quickly (i.e., less latency) and/or with a lower computational load. Using longer time constants may be more accurate or give more stable results. The threshold may be chosen using a calibration routine when the apparatus is started up which would have the advantage of tending to give higher accuracy but requires more time and complexity in the equipment. Alternatively, the threshold could be set either by design or at manufacturing time - which would be cheaper. This process serves to further reduce unwanted components.

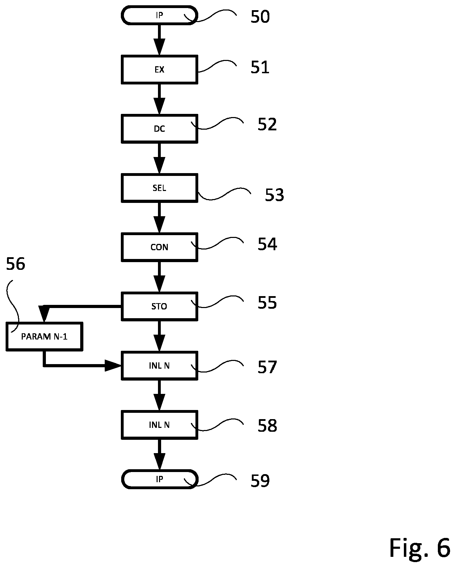

Fig. 6 represents the above described method in the form of a flow. At step 50 an input of the video sequence is received. At step 51, time varying signal segments are extracted. At step 52, the time varying signal segments are cleaned and the DC component removed. At step 53, a selection amongst the time-varying signal segments is made as described above. At step 54 the time varying signal segments are converted to a frequency or periodic representation and converted signal segments from a given time slot are combined. At step 55 the combined signal segments are stored. At step 56 interpolation parameters are derived from the combined signal segment and stored. At step 57, an interpolation of a current transformed signal segment is performed using previously stored parameters. At step 58 the interpolated stored signal segments are combined into a final signal. At step 59 a physiological information is extracted from the final signal.

The claims define the matter for which protection is sought in terms of the technical features of the invention.

## Claims

1. A computer implemented method of extracting a physiological information representing a vital sign of a subject, the method comprising:

    - receiving a sequence of video images of the subject (1);
    - for a defined time-period, extracting a plurality of signal segments (32) in a time-domain form from a plurality of respective patches in images in the sequence of video images;
    - selecting a subset of the plurality of signal segments (32) wherein selecting comprises rejecting segments having a high maximum signal level;
    - converting at least said subset to produce a plurality of transformed signal segments $(Ta_n, Tb_n)$ ;
    - combining the at least said subset of the plurality of transformed signal segments $(Ta_n, Tb_n)$ to produce a combined signal segment (41), and

    finding a dominant frequency or periodic component in the combined signal segment, the dominant frequency or periodic component being representative of the physiological information.

2. The method of claim 1 wherein selecting comprises rejecting segments having a maximum signal level greater than a threshold.

3. The method of claim 1 wherein selecting comprises weighting segments with weights having an inverse relationship to their maximum signal level.

4. The method of claim 2 wherein the inverse relationship is of the form of a power law or an inverse exponential.

5. The method of any of the previous claims wherein the conversion is performed using one of a power spectral density function, an auto-correlation function or a Fourier transform.

6. The method of any of the previous claims further comprising

- Repeating at least once the method of said previous claim;
- Storing the combined signal segment at each repetition to produce a plurality of combined signal segments, and

Averaging the plurality of combined signal segments to produce a final combined signal.

7. The method of claim 6 wherein the settings for the averaging of the plurality of combined signal segments are derived from a previously stored combined signal segment.

8. The method of claim 7 further comprising performing an interpolation of the combined signal.

9. The method of any previous claim further comprising the step of acquiring a sequence of video frames and wherein the extraction of the plurality of signal segments is performed using remote photoplethysmography.

10. The method of any previous claim comprising selecting at least one patch and extracting the plurality of segments in a time-domain form from the at least one patch.

11. A system for extracting a physiological information representative of a vital sign of a subject, the system comprising:

- An input (21) configured to receive a sequence of video images of the subject;
- A signal extraction unit (23) configured to, for a defined time-period, extract a plurality of signal segments in a time-domain form from a plurality of respective patches in images in the sequence of video images;
- A selection unit (25) configured to select a subset of the plurality of signal segments wherein selecting comprises rejecting segments having a high maximum signal level;
- A convertion unit (26) configured to convert at least said subset to produce a plurality of transformed signal segments;
- A combining unit (27) configured to combine the at least said subset of the plurality of transformed signal segments to produce a combined signal segment, and
- A physiological measurement extractor (28) configured to find a dominant frequency or periodic component in the combined signal segment, the dominat frequency or periodic component being representative of the physiological information.

12. The system of claim 11 wherein the combining unit further comprises a time-averaging unit configured to store the combined signal segments, to store interpolation settings, to apply those stored settings to interpolate subsequent combined signal segments and to combine the stored combined signal segments.

13. A computer program software product, stored on a computer-readable medium, configured, when executed on a processor, to execute the method of any of claims 1 to 10.

14. A physiological measurement equipment comprising a system according to any of claims 11 and 12.

**Patentansprüche**

1. Ein computergestütztes Verfahren zum Extrahieren physiologischer Informationen, die ein Vitalzeichen eines Subjekts darstellen, wobei das Verfahren Folgendes umfasst:

- Empfangen einer Sequenz von Videobildern des Subjekts (1);
- für einen definierten Zeitraum eine Vielzahl von Signalsegmenten (32) in einer Zeitbereichsform aus einer Vielzahl jeweiliger Bildfelder in der Sequenz von Videobildern zu extrahieren;
- Auswählen einer Teilmenge der Vielzahl von Signalsegmenten (32), wobei das Auswählen das Ablehnen von Segmenten mit einem hohen maximalen Signalpegel umfasst;
- Umwandeln von zumindest der Teilmenge, um eine Vielzahl transformierter Signalsegmente ($Ta_n$, $Tb_n$) zu erzeugen;
- Kombinieren der zumindest genannten Teilmenge der Vielzahl von transformierten Signalsegmenten ($Ta_n$,

$Tb_n$), um ein kombiniertes Signalsegment (41) zu erzeugen, und
- Finden einer dominanten Frequenz oder periodischen Komponente im kombinierten Signalsegment, wobei die dominante Frequenz oder periodische Komponente die physiologischen Informationen repräsentiert.

2. Das Verfahren nach Anspruch 1, wobei das Auswählen das Ablehnen von Segmenten umfasst, deren maximaler Signalpegel über einem Schwellenwert liegt.

3. Das Verfahren gemäß Anspruch 1, wobei das Auswählen das Gewichten von Segmenten mit Gewichten umfasst, die in einer umgekehrten Beziehung zu ihrem maximalen Signalpegel stehen.

4. Das Verfahren nach Anspruch 2, wobei die inverse Beziehung die Form einer Potenzfunktion oder einer inversen Exponentialfunktion hat.

5. Das Verfahren gemäß einem der vorhergehenden Ansprüche, wobei die Konvertierung unter Verwendung einer Leistungsdichtefunktion, einer Autokorrelationsfunktion oder einer Fourier-Transformation durchgeführt wird.

6. Das Verfahren eines der vorstehenden Ansprüche, weiter umfassend

   - Mindestens einmaliges Wiederholen des Verfahrens des vorhergehenden Anspruchs;
   - Speichern des kombinierten Signalsegments bei jeder Wiederholung, um eine Vielzahl von kombinierten Signalsegmenten zu erzeugen, und
   - Mitteln der Vielzahl kombinierter Signalsegmente zur Erzeugung eines endgültigen kombinierten Signals.

7. Das Verfahren gemäß Anspruch 6, wobei die Einstellungen für die Mittelwertbildung der Vielzahl kombinierter Signalsegmente aus einem zuvor gespeicherten kombinierten Signalsegment abgeleitet werden.

8. Das Verfahren nach Anspruch 7 umfasst außerdem das Durchführen einer Interpolation des kombinierten Signals.

9. Das Verfahren gemäß einem der vorhergehenden Ansprüche umfasst außerdem den Schritt des Erfassens einer Sequenz von Videobildern, wobei die Extraktion der Vielzahl von Signalsegmenten unter Verwendung einer Fern-Photoplethysmographie durchgeführt wird.

10. Das Verfahren gemäß einem der vorhergehenden Ansprüche umfasst das Auswählen von mindestens einem Patch und das Extrahieren der Vielzahl von Segmenten in einer Zeitbereichsform aus dem mindestens einen Patch.

11. Ein System zum Extrahieren physiologischer Informationen, die ein Vitalzeichen eines Subjekts darstellen, wobei das System Folgendes umfasst:

    - Einen Eingang (21), der zum Empfangen einer Sequenz von Videobildern des Subjekts konfiguriert ist;
    - Eine Signalextraktionseinheit (23), die dazu konfiguriert ist, für einen definierten Zeitraum eine Vielzahl von Signalsegmenten in einer Zeitbereichsform aus einer Vielzahl jeweiliger Patches in Bildern in der Sequenz von Videobildern zu extrahieren;
    - Eine Auswahleinheit (25), die dazu konfiguriert ist, eine Teilmenge der Vielzahl von Signalsegmenten auszuwählen, wobei das Auswählen das Ablehnen von Segmenten mit einem hohen maximalen Signalpegel umfasst;
    - Eine Konvertierungseinheit (26), die dazu konfiguriert ist, zumindest die Teilmenge umzuwandeln, um eine Vielzahl transformierter Signalsegmente zu erzeugen;
    - Eine Kombinationseinheit (27), die dazu konfiguriert ist, die mindestens genannte Teilmenge der Vielzahl von transformierten Signalsegmenten zu kombinieren, um ein kombiniertes Signalsegment zu erzeugen, und
    - Einen Physiologischer Messwert-Extraktor (28), der dazu konfiguriert ist, eine dominante Frequenz oder periodische Komponente im kombinierten Signalsegment zu finden, wobei die dominante Frequenz oder periodische Komponente die physiologischen Informationen repräsentiert.

12. Das System nach Anspruch 11, wobei die Kombinationseinheit außerdem eine Zeitmittelungseinheit umfasst, die zum Speichern der kombinierten Signalsegmente, zum Speichern von Interpolationseinstellungen, zum Anwenden dieser gespeicherten Einstellungen zum Interpolieren nachfolgender kombinierter Signalsegmente und zum Kombinieren der gespeicherten kombinierten Signalsegmente konfiguriert ist.

13. Ein Computerprogramm-Softwareprodukt, das auf einem computerlesbaren Medium gespeichert ist und bei Ausführung auf einem Prozessor so konfiguriert ist, dass es das Verfahren nach einem der Ansprüche 1 bis 10 ausführt.

**14.** Eine physiologische Messvorrichtung, die ein System gemäß einem der Ansprüche 11 und 12 umfasst.

## Revendications

**1.** Procédé mis en oeuvre par ordinateur d'extraction d'une information physiologique représentant un signe vital d'un sujet, le procédé comprenant :

- la réception d'une séquence d'images vidéo du sujet (1) ;
- pendant une période de temps définie, l'extraction d'une pluralité de segments de signal (32) sous une forme de domaine temporel à partir d'une pluralité de fragments respectifs dans des images dans la séquence d'images vidéo ;
- la sélection d'un sous-ensemble de la pluralité de segments de signal (32), dans lequel la sélection comprend le rejet de segments présentant un niveau de signal maximum élevé ;
- la conversion d'au moins ledit sous-ensemble pour produire une pluralité de segments de signal transformés $(Ta_n, Tb_n)$;
- la combinaison du au moins ledit sous-ensemble de la pluralité de segments de signal transformés $(Ta_n, Tb_n)$ pour produire un segment de signal combiné (41), et
- la découverte d'une fréquence dominante ou d'une composante périodique dans le segment de signal combiné, la fréquence dominante ou la composante périodique étant représentative de l'information physiologique.

**2.** Procédé selon la revendication 1, dans lequel la sélection comprend le rejet de segments présentant un niveau de signal maximum supérieur à un seuil.

**3.** Procédé selon la revendication 1, dans lequel la sélection comprend une pondération de segments avec des poids présentant une relation inverse par rapport à leur niveau de signal maximum.

**4.** Procédé selon la revendication 2, dans lequel la relation inverse est sous la forme d'une loi de puissance ou d'une exponentielle inverse.

**5.** Procédé selon l'une quelconque des revendications précédentes, dans lequel la conversion est effectuée à l'aide de l'une d'une fonction de densité spectrale de puissance, d'une fonction d'autocorrélation ou d'une transformée de Fourier.

**6.** Procédé selon l'une quelconque des revendications précédentes, comprenant en outre :

- la répétition au moins une fois du procédé de ladite revendication précédente ;
- le stockage du segment de signal combiné à chaque répétition pour produire une pluralité de segments de signal combinés, et
- le calcul de la moyenne de la pluralité de segments de signal combinés pour produire un signal combiné final.

**7.** Procédé selon la revendication 6, dans lequel les réglages pour le calcul de la moyenne de la pluralité de segments de signal combinés sont dérivés d'un segment de signal combiné stocké précédemment.

**8.** Procédé selon la revendication 7, comprenant en outre la réalisation d'une interpolation du signal combiné.

**9.** Procédé selon l'une quelconque des revendications précédentes, comprenant en outre l'étape d'acquisition d'une séquence d'images vidéo et dans lequel l'extraction de la pluralité de segments de signal est effectuée à l'aide d'une photopléthysmographie à distance.

**10.** Procédé selon l'une quelconque des revendications précédentes, comprenant la sélection d'au moins un fragment et l'extraction de la pluralité de segments sous une forme de domaine temporel à partir du au moins un fragment.

**11.** Système pour extraire une information physiologique représentative d'un signe vital d'un sujet, le système comprenant :

- une entrée (21) configurée pour recevoir une séquence d'images vidéo du sujet ;
- une unité d'extraction de signal (23) configurée, pendant une période de temps définie, pour extraire une pluralité de segments de signal sous une forme de domaine temporel à partir d'une pluralité de fragments respectifs dans des images dans la séquence d'images vidéo ;
- une unité de sélection (25) configurée pour sélectionner un sous-ensemble de la pluralité de segments de signal, dans lequel la sélection comprend le rejet de segments présentant un niveau de signal maximum élevé
- une unité de conversion (26) configurée pour convertir au moins ledit sous-ensemble pour produire une pluralité de segments de signal transformés ;
- une unité de combinaison (27) configurée pour combiner le au moins dit un sous-ensemble de la pluralité de segments de signal transformés pour produire un segment de signal combiné, et
- un extracteur de mesure physiologique (28) configuré pour trouver une fréquence dominante ou une composante périodique dans le segment de signal combiné, la fréquence dominante ou la composante périodique étant représentative de l'information physiologique.

12. Système selon la revendication 11, dans lequel l'unité de combinaison comprend en outre une unité de calcul de moyenne temporelle configurée pour stocker les segments de signal combinés, pour stocker des réglages d'interpolation, pour appliquer ces réglages stockés pour interpoler de prochains segments de signal combinés et pour combiner les segments de signal combinés stockés.

13. Produit logiciel de programme informatique, stocké sur un support lisible par ordinateur, configuré, lorsqu'il est exécuté sur un processeur, pour exécuter le procédé selon l'une quelconque des revendications 1 à 10.

14. Équipement de mesure physiologique comprenant un système selon l'une quelconque des revendications 11 et 12.

Fig 1

Fig 2

Fig 3

Fig 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Estimation of Respiratory Pattern From Video Using Selective Ensemble Aggregation. **PRATHOSH A P et al.** IEEE TRANSACTIONS ON SIGNAL PROCESSING. IEEE SERVICE CENTER, 02 February 2017, vol. 65, 2902-2916

- **VIOLA, P. ; JONES, M.J.** Robust real-time object detection. *Proc. of IEEE workshop on statistical and computational theories of vision,* 13 July 2001